(19) **Europäisches Patentamt European Patent Office Office européen des brevets**

(11) **EP 2 335 553 A1**

(12) **EUROPEAN PATENT APPLICATION**
published in accordance with Art. 153(4) EPC

(43) Date of publication:
**22.06.2011 Bulletin 2011/25**

(51) Int Cl.:
***A61B 1/00*** *(2006.01)*

(21) Application number: **10783225.5**

(86) International application number:
**PCT/JP2010/057456**

(22) Date of filing: **27.04.2010**

(87) International publication number:
**WO 2010/140441 (09.12.2010 Gazette 2010/49)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA ME RS**

(30) Priority: **01.06.2009 JP 2009132390**

(71) Applicant: **Olympus Medical Systems Corp. Tokyo 151-0072 (JP)**

(72) Inventors:
• **IKUMA Soichi**
  **Tokyo 151-0072 (JP)**
• **KAWASHIMA, Tomonao**
  **Tokyo 151-0072 (JP)**

(74) Representative: **Gunzelmann, Rainer**
  **Wuesthoff & Wuesthoff**
  **Patent- und Rechtsanwälte**
  **Schweigerstraße 2**
  **81541 München (DE)**

(54) **MEDICAL EQUIPMENT SYSTEM AND METHOD FOR CALIBRATING MEDICAL INSTRUMENT**

(57)    An endoscope system 1 is provided with an insertion portion 12 having a rigid portion 13 at a distal end portion of the insertion portion and a channel 14 passed through inside of the rigid portion, an ultrasound probe 21 that has a first sensor 40 for detecting a position and a direction at a probe distal end portion 22, is inserted from an insertion port 14A on a proximal end portion side of a channel 14 and projects from a projection port 14B of the rigid portion 13, a position calculation section 31A that calculates a position and a direction of the first sensor 40, and a direction calculation section 31B that calculates a direction of the probe distal end portion 22 based on a positional information variation of the first sensor 40 before and after linear movement of the probe distal end portion 22.

# FIG.8

**Description**

Technical Field

The present invention relates to a medical equipment system provided with a medical instrument used while projecting from a distal end of an insertion portion of an endoscope inserted into the body of a subject and a medical instrument calibration method, and more particularly, to a medical equipment system and a medical instrument calibration method capable of detecting a precise direction of the distal end portion of the medical instrument.

Background Art

In recent years, an insertion navigation system is disclosed which forms a three-dimensional image of a tube, for example, the bronchus of the lung from three-dimensional image data of a subject obtained using a CT apparatus, determines a path up to a target point along the tube on the three-dimensional image and further forms a virtual endoscope image of the tube along the path based on the three-dimensional image data. Using, for example, an insertion navigation system disclosed in Japanese Patent Application Laid-Open Publication No. 2004-180940 allows an operator to correctly guide the distal end of the insertion portion of an endoscope to the vicinity of a region of interest in a short time. However, there is a limit to the thickness, that is, the diameter, of the tube through which the insertion portion can be inserted, and the insertion portion cannot be inserted up to the periphery of the bronchus. For this reason, after the distal end of the insertion portion reaches the vicinity of the region of interest, by causing a medical instrument such as treatment instrument or ultrasound probe of a smaller diameter to project from the distal end of the insertion portion, the operator can extract a sample of the region of interest or photograph an ultrasound image of target tissue.

To photograph an ultrasound image of target tissue or extract a sample of the region of interest, it is necessary to detect the position and direction of the distal end portion of the medical instrument. Japanese Patent Application Laid-Open Publication No. 2006-223849 and Japanese Patent Application Laid-Open Publication No. 2007-130154 propose a method of arranging a sensor at the distal end portion of the medical instrument to detect the position and direction of the distal end portion of a medical instrument.

In some cases, when an ultra-small sensor to be attached to a distal end portion of a medical instrument with a small diameter had bad attachment accuracy, it was disabled to detect with desired accuracy the position and direction, in particular the direction, of the distal end portion of the medical instrument.

The present invention has an objective to provide a medical equipment system which includes a medical instrument used while projecting from a distal end of an insertion portion and which performs highly accurate inspection or treatment, and a medical instrument calibration method which performs highly accurate inspection or treatment.

Disclosure of Invention

Means for Solving the Problem

To realize the above-mentioned objective, a medical equipment system according to an embodiment of the present invention is provided with insertion means having a rigid portion configuring a distal end portion of an insertion portion and a channel passed through inside of the rigid portion, a medical instrument whose medical instrument distal end portion projects from a projection port of the rigid portion, the medical instrument being inserted from an insertion port on a proximal end portion side of the channel, and direction calculation means that calculates a longitudinal direction of the medical instrument distal end portion based on a positional variation of the medical instrument distal end portion inserted into the channel.

Furthermore, a medical instrument calibration method according to another embodiment of the present invention includes an insertion step of inserting the medical instrument from an insertion port on a proximal end portion side of a channel of insertion means having a rigid portion configuring a distal end portion of an insertion portion and the channel passed through inside of the rigid portion, a first calculation step of calculating the position of the medical instrument distal end portion in a first place based on information of a first sensor disposed at the medical instrument distal end portion, capable of detecting a position and a direction, a probe moving step of moving the position of the medical instrument distal end portion from the first place to a second place on a straight line, a second calculation step of calculating the position of the medical instrument distal end portion in the second place, and a distal end portion direction calculation step of calculating the direction of the medical instrument distal end portion based on the position calculated in the first calculation step and the position calculated in the second calculation step.

Brief Description of the Drawings

Fig. 1 is a diagram illustrating a situation in which a region of interest of the lung of a subject is being inspected using an endoscope system according to a first embodiment;
Fig. 2 is a configuration diagram illustrating a configuration of the endoscope system of the first embodiment;
Fig. 3 is a schematic cross-sectional view illustrating an ideal structure of an ultrasound probe which is a medical instrument of the endoscope system of the first embodiment;
Fig. 4 is a schematic cross-sectional view illustrating an example of an actual structure of the ultrasound probe which is a medical instrument of the endoscope system of the first embodiment;
Fig. 5 is a configuration diagram illustrating a configuration of a navigation unit of the endoscope system of the first embodiment;
Fig. 6 is a flowchart illustrating a processing flow of the medical system of the first embodiment;
Fig. 7 is a schematic cross-sectional view illustrating operation of the medical system of the first embodiment;
Fig. 8 is a cross section schematic diagram illustrating the operation of the medical system of the first embodiment;
Fig. 9 is a schematic cross-sectional view illustrating the operation of the medical system of the first embodiment;
Fig. 10 is a schematic cross-sectional view illustrating operation of a medical system according to a second embodiment;
Fig. 11 is a schematic cross-sectional view illustrating the operation of the medical system of the second embodiment;
Fig. 12 is a schematic cross-sectional view illustrating the operation of the medical system of the second embodiment;
Fig. 13 is a display screen illustrating an example of image processing of a monitor illustrating an endoscope system according to a third embodiment of the present invention;
Fig. 14 is a flowchart illustrating a processing flow of the medical system of the third embodiment;
Fig. 15 is a schematic cross-sectional view of an endoscope illustrating an endoscope system according to a fourth embodiment;
Fig. 16 is a schematic cross-sectional view of the endoscope illustrating the endoscope system of the fourth embodiment;
Fig. 17 is a configuration diagram illustrating a configuration of the endoscope system of the fourth embodiment;
Fig. 18A is a diagram illustrating a coordinate system in the endoscope system of the fourth embodiment;
Fig. 18B is a diagram illustrating the coordinate system in the endoscope system of the fourth embodiment; and
Fig. 18C is a diagram illustrating the coordinate system in the endoscope system of the fourth embodiment.

Best Mode for Carrying Out the Invention

<First embodiment>

Hereinafter, an endoscope system 1, which is a medical equipment system according to a first embodiment of the present invention, and a calibration method of an ultrasound probe (hereinafter also simply referred to as "probe") 21 of a small diameter, which is a medical instrument, will be described with reference to the accompanying drawings.

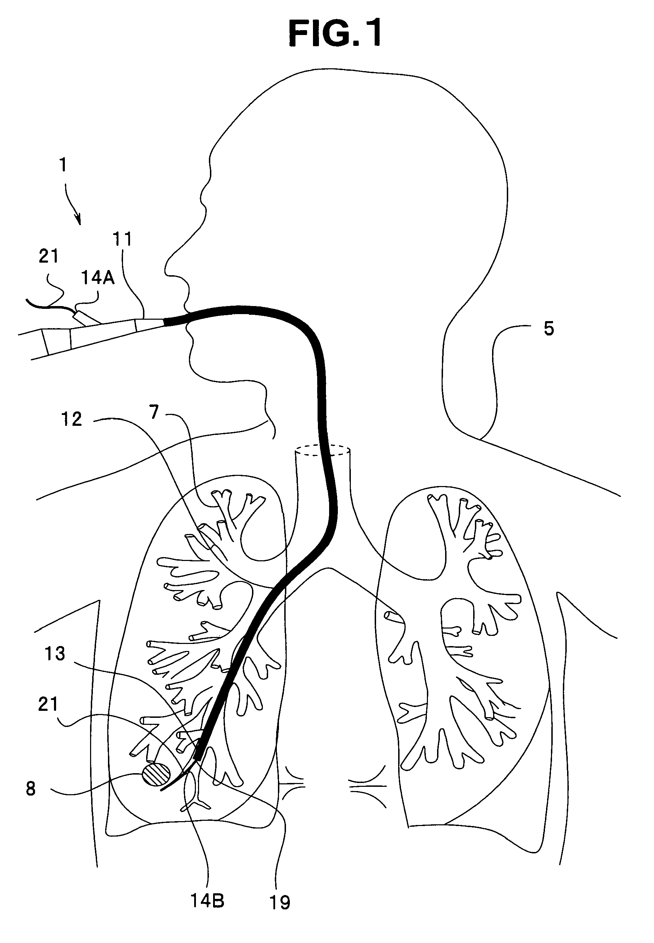

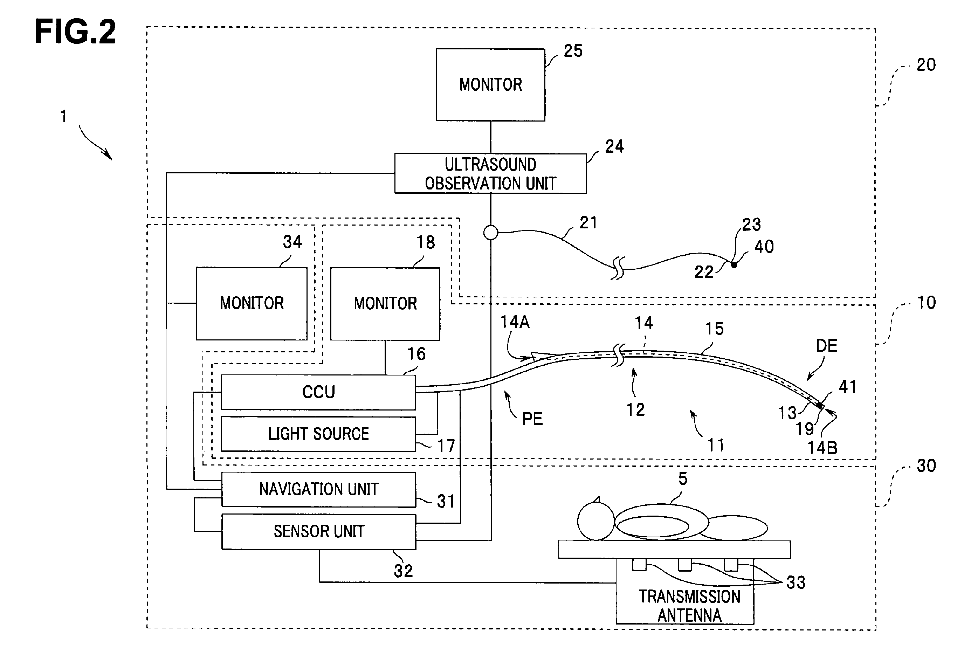

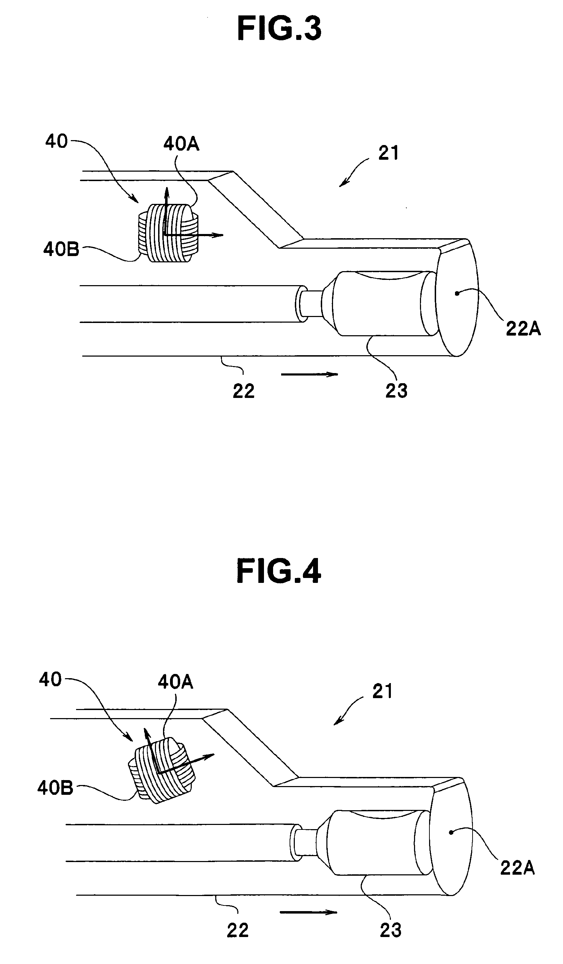

Fig. 1 is a diagram illustrating a situation in which a region of interest of the lung of a subject is being inspected using an endoscope system according to the first embodiment of the present invention, Fig. 2 is a configuration diagram illustrating a configuration of the endoscope system of the present embodiment, Fig. 3 and Fig. 4 are schematic cross-sectional views illustrating a structure of a probe, which is a medical instrument of the endoscope system of the present embodiment.
Fig. 1 shows a situation in which a rigid portion 13 making up an endoscope distal end portion, which is an insertion portion distal end portion of an insertion portion 12 of an endoscope apparatus 10 of the endoscope system 1 is inserted into a tube of a minimum diameter of the bronchus 7 of the subject 5 up to which insertion is possible. A probe distal end portion of the ultrasound probe (hereinafter also referred to as "probe") 21 which is a medical instrument inserted into a channel 14 (see Fig. 2) from a projection port 14B on the proximal end portion side projects from the projection port 14B of the rigid portion 13 and inspects tissue of a region of interest 8.
As shown in Fig. 1, the insertion portion 12 of the endoscope apparatus 10 is as thin as on the order of diameter 3 mm so as to be insertable into a thin bronchus tube cavity, but the probe 21 is, for example, on the order of diameter 1 mm so as to be insertable into the thinner peripheral bronchus tube cavity. Since the region of interest 8 is within the thin peripheral bronchus, it often cannot be recognized using a CCD 19 or the like disposed in the rigid portion 13.
Next, as shown in Fig. 2, the endoscope system 1 is provided with the endoscope apparatus 10, which is insertion means, an ultrasound observation apparatus 20 and a navigation apparatus 30. The endoscope apparatus 10 includes an endoscope 11 having the CCD 19 which is image pickup means in the rigid portion 13 of the insertion portion 12 having a flexible portion 15 and the rigid portion 13, a light source 17 that supplies illumination light to the endoscope 11, a CCU (camera control unit) 16 that controls the CCD 19 which is image pickup means and processes an image signal obtained from the CCD 19 into a video signal and a monitor 18 that displays an endoscope image. The channel 14 having openings at the insertion port 14A on a proximal end portion side (PE) and at the projection port 14B of the rigid portion 13 on the endoscope distal end portion side (DE) passes through the insertion portion 12. While the flexible portion 15 is flexible, the rigid portion 13 is not flexible.

The ultrasound observation apparatus 20 has a probe 21 having an ultrasound transducer 23 at a probe distal end portion (hereinafter also simply referred to as "distal end portion") 22, which is a medical instrument distal end portion, an ultrasound observation unit 24 that controls the ultrasound transducer 23 and processes an ultrasound signal obtained and a monitor 25 that displays an ultrasound image.

The navigation apparatus 30 has transmission antennas 33 which are magnetic field generating means for generating magnetic fields for a first sensor 40 disposed at the distal end portion 22 and a second sensor 41 disposed in the rigid portion 13 of the insertion portion 12 to detect the position and direction, a sensor unit 32 that processes output data of the first sensor 40 and the second sensor 41, a navigation unit 31 that calculates positions and directions of the distal end portion 22 of the probe 21 and the distal end of the insertion portion 12 based on information of the sensor unit 32 and is insertion supporting means for supporting the insertion operation, and a monitor 34 that performs display for navigation. The sensor unit 32 and the navigation unit 31 need not be independent units, but may be integrated in one single unit.

The sensor unit 32 shown in Fig. 2 sends an AC current to coils (not shown) located at a plurality of different positions in the transmission antennas 33 and the transmission antennas 33 generate AC magnetic fields. The first sensor 40 and the second sensor 41 detect the AC magnetic fields from the transmission antennas 33 and can detect the position and direction based on the detected magnetic field strength.

That is, as shown in Fig. 3 and Fig. 4, the first sensor 40 is a magnetic field detection sensor that has, for example, two coils 40A and 40B that detect magnetic fields in directions orthogonal to each other. That is, a coil axis which is a magnetic field detection direction of the coil 40A is orthogonal to a coil axis which is a magnetic field detection direction of the coil 40B.

Therefore, the first sensor 40 can detect distances from and directions of the respective coils located at a plurality of different positions in the transmission antennas 33. Thus, the sensor unit 32 can detect a position (x, y, z) and a direction ($\alpha$, $\beta$, $\gamma$) of the first sensor 40 using the positions of the transmission antennas 33 as references, that is, parameters of six degrees of freedom. The sensor position is, for example, three-dimensional coordinate values of the coil center point of the coils 40A and 40B and the sensor direction is the direction of, for example, the coil axis of the coil 40A.

As shown in Fig. 2, the second sensor 41 disposed at the rigid portion 13 is a magnetic field detection sensor that has a structure similar to that of the first sensor 40, that is, having two coils that detect magnetic fields in directions orthogonal to each other. The coil axis which is the magnetic field detection direction of one coil of the second sensor 41 is parallel to the longitudinal direction of the elongated distal end portion 22 (rigid portion 13) and the coil axis which is the magnetic field detection direction of the other coil is parallel to the vertical direction of the endoscope image out of the directions orthogonal to the longitudinal directions of the distal end portion 22. Hereinafter, when the sensor direction is indicated, suppose the direction substantially parallel to the longitudinal direction of the distal end portion 22 will be referred to as an "axial direction" and the direction substantially orthogonal to the longitudinal direction will be referred to as a "radial direction."

The sensor unit 32 detects the positions and directions of the first sensor 40 and the second sensor 41 and calculates the position and direction of the ultrasound transducer 23 disposed at the distal end portion 22. The navigation unit 31 then performs navigation based on the positions and directions of the ultrasound transducer 23 and the distal end portion 22 calculated by the sensor unit 32. The position of the ultrasound transducer 23 is, for example, the center position of the ultrasound transducer 23, the direction thereof is a direction orthogonal to the direction in which ultrasound is generated, and the position of the distal end portion 22 is the center position of the distal end face of the probe 21 and the direction thereof is the longitudinal direction of the elongated distal end portion 22.

However, as shown in Fig. 3, when a smaller magnetic field sensor is disposed at the distal end portion 22 of the probe 21 of a small diameter, it is ideal that the magnetic field detection direction of the coil 40A be disposed so as to be parallel to the longitudinal direction of the distal end portion 22, but this is not easy. That is, as shown in Fig. 4, the magnetic field detection direction of the coil 40A may be actually not parallel to the longitudinal direction of the distal end portion 22. Fig. 4 shows an example where the magnetic field detection direction of the coil 40A is exaggeratedly deviated from the longitudinal direction of the distal end portion 22 for ease of explanation.

As shown in Fig. 4, when the magnetic field detection direction of the coil 40A does not coincide with the longitudinal direction of the distal end portion 22, there is an error between the magnetic field detection direction of the coil 40A calculated by the sensor unit 32 and the longitudinal direction of the distal end portion 22. However, as will be described later, the endoscope system 1 can calibrate the probe 21, and can thereby calculate the direction with high accuracy.

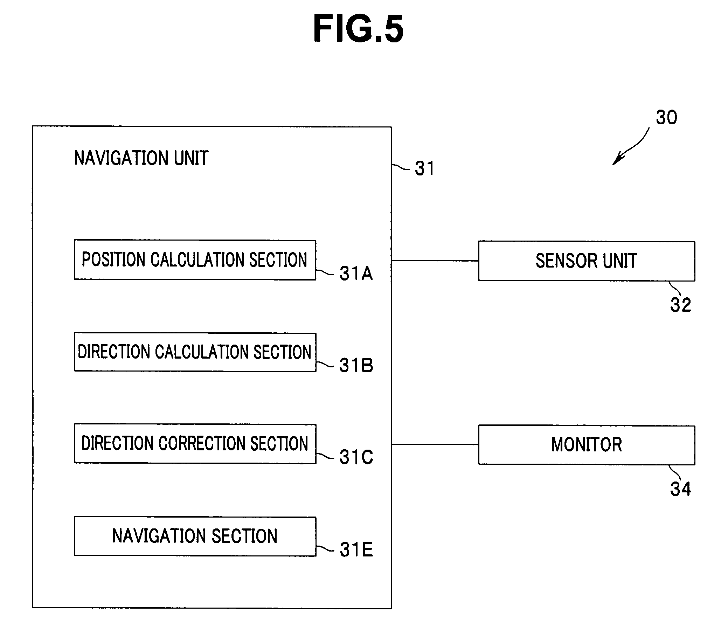

Here, Fig. 5 is a configuration diagram illustrating a configuration of a navigation unit 31 of the endoscope system 1 of the present embodiment. As shown in Fig. 5, the navigation unit 31 includes a position calculation section 31A which is position calculation means for calculating the position and direction of the first sensor 40 from information of the first sensor 40, a direction calculation section 31 B which is direction calculation means for calculating the longitudinal direction of the distal end portion 22, a direction correction section 31 C which is direction correction means for correcting the direction detected by the first sensor 40, and a navigation section 31E which is navigation means for performing navigation that inserts the distal end portion 22 up to the region of interest 8 based on the position of the distal end portion 22. As has already been described, since the region of interest 8 is located in the small peripheral bronchus, the region of interest 8 may not always be recognized using the CCD 19 or the like disposed at the rigid portion 13.

As will be described later, the direction calculation section 31B calculates the longitudinal direction of the distal end portion 22 based on the place to which the distal end portion 22, that is, the first sensor 40 moves on a straight line, for example, the position of the first sensor 40 before and after the movement when the channel 14 in the rigid portion 13 is moved, and thereby calculates the amount of difference between the magnetic field detection direction of the coil 40A and the longitudinal direction of the distal end portion 22, the direction correction section 31C corrects the direction detected by the first sensor 40 and calculates the longitudinal direction of the distal end portion 22, and the endoscope system 1 can thereby calculate the direction with high accuracy.

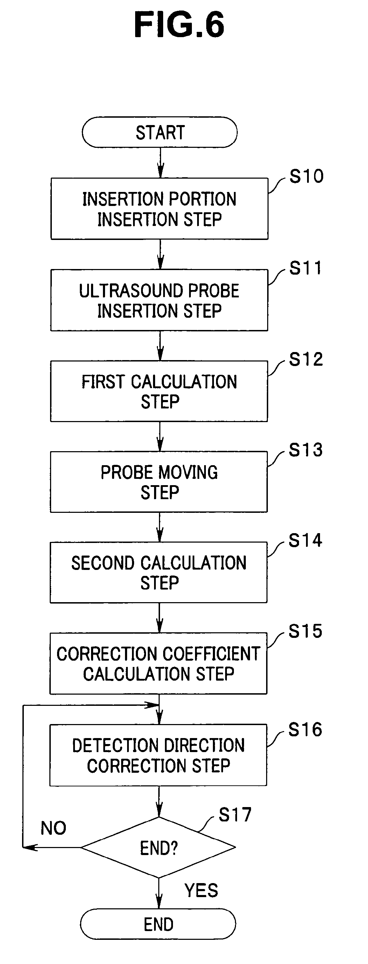

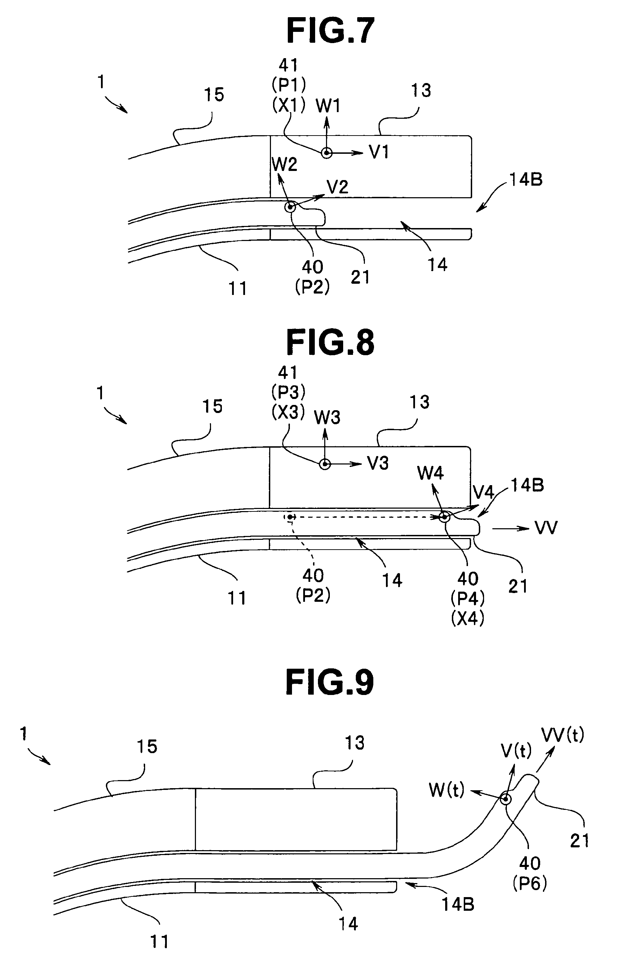

Here, the operation of the endoscope system 1 will be described using Fig. 6, Fig. 7, Fig. 8 and Fig. 9. Fig. 6 is a flowchart illustrating a processing flow of the medical system of the present embodiment and Fig. 7 to Fig. 9 are schematic cross-sectional views illustrating the operation of the medical system according to the present embodiment. Hereinafter, the processing flow of the endoscope system 1 of the present embodiment will be described according to the flowchart in Fig. 6.

<Step S 1 0> Insertion portion insertion step

The operator inserts the insertion portion 12 of the endoscope apparatus 10 into the bronchus 7 of the subject 5. In that case, by forming a virtual endoscope image of the bronchus 7 using a publicly known insertion navigation system based on the three-dimensional image data and performing insertion support, the operator can correctly guide the distal end of the insertion portion 12 to the vicinity of the region of interest 8 in a short time.

<Step S11> Probe insertion step

As shown in Fig. 7, the operator inserts the probe 21 from the insertion port 14A of the channel 14 of the insertion portion 12 so that the first sensor 40 is located at the position P2 close to the proximal end portion in the channel 14 of the rigid portion 13.

<Step S 12> First calculation step

The operator instructs the navigation unit 31 on the first direction correction processing.
Upon receiving the instruction on the first direction correction processing, the navigation unit 31 acquires data (position and direction) of the first sensor 40 and the second sensor 41 from the sensor unit 32.
In this case, suppose the position data of the second sensor 41 is P1, the axial direction data is vector V1, radial direction data is vector W1, the magnetic field detection direction data of the coil 40A of the first sensor 40 is vector V2, and the magnetic field detection direction data of the coil 40B is vector W2.

<Step S13> Probe moving step

Next, as shown in Fig. 8, the operator moves the position of the probe 21 with respect to the rigid portion 13 to the distal end direction P4 within a range in which the first sensor 40 of the probe 21 is located in the channel 14 of the rigid portion 13. Since the channel 14 in the rigid portion 13 is linear, the distal end portion 22, that is, the first sensor 40 moves on a straight line.

<Step S14> Second calculation step

The operator instructs the navigation unit 31 on second direction correction processing.
Upon receiving the instruction of the second direction correction processing, the navigation unit 31 acquires data (position and direction) of the second sensor 41 and data (position and direction) of the first sensor 40 form the sensor unit.
In this case, suppose the position data of the second sensor 41 is P3, the axial direction data is vector V3, radial direction data is vector W3, magnetic field detection direction data from the coil 40A of the first sensor 40 is vector V4, and magnetic field detection direction data from the coil 40B is vector W4.

<Step S15> Correction coefficient calculation step

Assuming that the moving direction of the probe 21 coincides with the longitudinal direction of the distal end portion 22 of the probe 21, the navigation unit 31 estimates the longitudinal direction of the distal end portion 22 of the probe 21. However, while the probe 21 is moving, the endoscope 11 may move due to movement, breathing or heart beat of the subject. To cancel out the movement of the endoscope 11, it is preferable to calculate the moving direction of the probe 21 as the longitudinal direction of the distal end portion 22 of the probe 21 based on the relative position of the probe 21 with respect to the endoscope 11.
The method of calculating the longitudinal direction VV of the distal end portion 22 will be described in (Equation 1) to (Equation 6) described below.
First, the navigation unit 31 sets vector X1, vector X3 and vector X4 as (Equation 1), (Equation 2) and (Equation 3) below respectively.

$$X1 = V1 \times W1 \text{ (vector product)} \quad \text{(Equation 1)}$$

$$X3 = V3 \times W3 \text{ (vector product)} \quad \text{(Equation 2)}$$

$$X4 = V4 \times W4 \text{ (vector product)} \quad \text{(Equation 3)}$$

Next, assuming the relative position of the probe 21 with respect to the endoscope 11 in the first calculation step before moving the probe is vector P1P2 from P1 to P2, relative position coefficients a, b and c are calculated when expressed by (Equation 4) below using V1, W1 and X1.

$$P2P1 = aV1 + bW1 + cX1 \quad \text{(Equation 4)}$$

Likewise, assuming the relative position of the probe 21 relative to the endoscope 11 in the second calculation step after moving the probe is vector P1P2 from P3 to P4, relative position coefficients $a_1$, $b_1$ and $c_1$ are calculated when expressed by (Equation 5) below using V3, W3 and X3.

$$P4P3 = a_1V3 + b_1W3 + c_1X3 \quad \text{(Equation 5)}$$

The axial direction of the probe 21 in the second calculation step, that is, the longitudinal direction VV of the distal end portion 22 is calculated from the moving direction of the probe 21, and is calculated based on the relative position with respect to the endoscope 11. Thus, VV can be calculated as expressed in (Equation 6) below using a, b, c, $a_1$, $b_1$ and $c_1$ which are relative position coefficients.

$$VV = P3P4 - P1P2 = (a_1-a)V3 + (b_1-b)W3 + (c_1-c)X3 \quad \text{(Equation 6)}$$

The longitudinal direction VV of the distal end portion 22 of the probe 21 calculated here is expressed as a function of magnetic field detection direction data of the coil 40A which is output data of the first sensor 40 and magnetic field detection direction data of the coil 40B. By expressing VV with this function, the output data of the first sensor 40 of the probe 21 is corrected and a correction coefficient for accurately calculating the longitudinal direction of the distal end portion 22 is calculated.
When VV is expressed as a function of V4, W4 and X4, VV is expressed by (Equation 7) below and the navigation unit 31 can calculate $a_2$, $b_2$ and $c_2$ which are correction coefficients using (Equation 6) and (Equation 7).

$$VV=a_2V4+b_2W4+c_2X4 \qquad \text{(Equation 7)}$$

<Step S 16> Detection direction correction step (navigation step)

The navigation apparatus 30 changes the navigation target from the rigid portion 13 of the insertion portion 12 to the distal end portion 22 of the probe 21. The navigation apparatus 30 creates navigation information based on the corrected longitudinal direction VV(t) of the distal end portion 22 and the detected position of the distal end portion 22. The operator inserts the probe 21 up to the vicinity of the region of interest 8 according to navigation information of the navigation apparatus 30 and performs observation using the ultrasound transducer 23.

As shown in Fig. 9, a longitudinal direction VV(t) of the distal end portion 22 at an arbitrary time t during navigation is calculated according to the following (Equation 8) based on the magnetic field detection direction data V(t) of the coil 40A which is the output data of the first sensor 40 at the arbitrary time t, the magnetic field detection direction data W(t) of the coil 40B and a2, b2 and c2 which are correction coefficients calculated in step S 15.

$$VV(t)=a_2V(t)+b_2W(t)+c_2(V(t)\times W(t)) \qquad \text{(Equation 8)}$$

<Step S17> End instruction

The navigation apparatus 30 continues the navigation until the operator sends an end instruction.

The correction coefficients a2, b2 and c2 used by the direction correction section 31C for correction are values specific to the probe 21. Therefore, the navigation apparatus 30 also has a storage section that stores the relationship between the probe whose correction coefficient is calculated once, in other words, the calibrated probe and the correction coefficient, and it is also possible to preferably use an endoscope system that informs the operator that the correction coefficient has already been calculated when the probe stored in the storage section is used.

An example has been described above where the position of the distal end portion 22 is corrected based on information of the second sensor 41. Even when the relative position of the bronchus 7 with respect to the region of interest 8 of the distal end portion 22 does not change, the position of the distal end portion 22 changes due to breathing or the like of the subject 5. However, in the case of movement of the distal end portion 22 due to breathing or the like of the subject 5, it is possible to assume that the second sensor 41 also simultaneously moves by the same amount. Thus, by correcting the position of the distal end portion 22 based on the information of the second sensor 41, it is possible to estimate the movement due to breathing or the like of the subject 5 and calculate the position of the distal end portion 22 more accurately.

When the region of interest 8 is located at a region where there is little influence of breathing or the like of the subject, the position of the distal end portion 22 need not be corrected based on the information of the second sensor 41. In other words, the second sensor 41 is unnecessary.

Although the ultrasound probe 21 has been illustrated above as an example of the medical instrument, the medical instrument is not limited to this, but a treatment instrument such as puncture needle, brush or forceps whose distal end is suitable for sampling of tissue may be used as the medical instrument.

As described so far, in the endoscope system 1 which is the medical equipment system of the present embodiment, when the first sensor 40 is disposed at the probe 21, even if the probe 21 is not disposed accurately, the probe 21, which is the medical instrument, calibrates the probe 21, and can thereby detect a precise longitudinal direction of the distal end portion 22. Thus, the endoscope system 1 can perform high accuracy inspection or treatment.

Furthermore, a magnetic field sensor made up of two coils whose coil axes are orthogonal to each other as the first sensor 40 and second sensor 41 has been illustrated in the present embodiment, but these need not be orthogonal to each other as long as the coil axis directions of the two coils are different. Furthermore, the magnetic field sensor may be made up of three or more coils or may be an MR sensor, MI sensor, FG sensor or the like.

<Second embodiment>

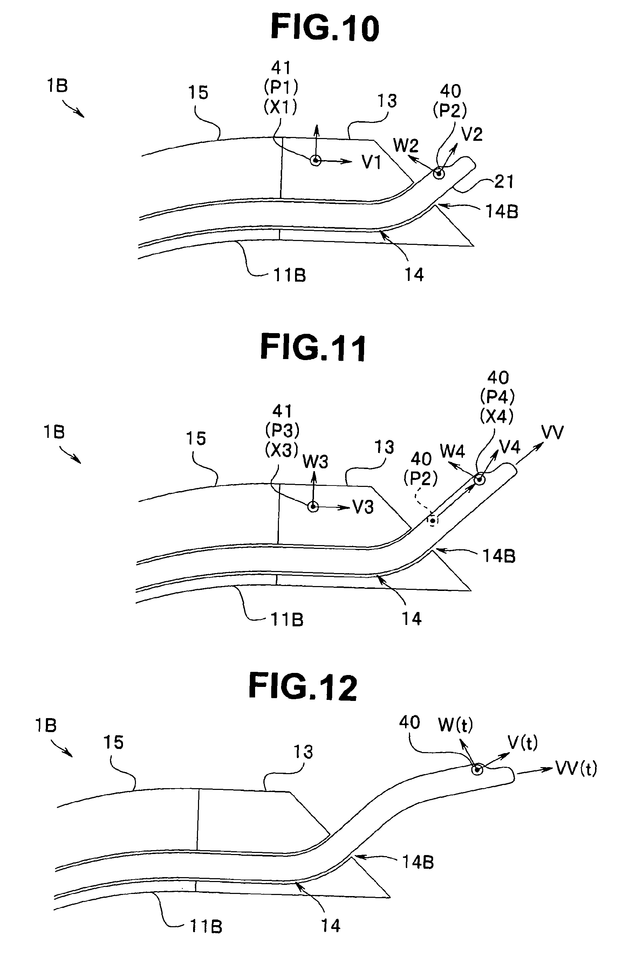

Hereinafter, an endoscope system 1B which is a medical equipment system according to a second embodiment of the present invention will be described with reference to the accompanying drawings. The endoscope system 1B of the present embodiment is similar to the endoscope system 1 of the first embodiment, and therefore the same components will be assigned the same reference numerals and descriptions thereof will be omitted. Fig. 10, Fig. 11 and Fig. 12 are schematic cross-sectional views illustrating the operation of the endoscope system of the second embodiment.

As shown in Fig. 10, since the endoscope 11 of the endoscope system 1 B of the present embodiment has a structure in which the probe 21 projects in a diagonal direction, the linear region of the channel 14 in the rigid portion 13 is short. For this reason, it is not easy to calibrate the probe 21 in the rigid portion 13.

However, as shown in Fig. 11, while the amount of projection is small even after projecting from the projection port 14B, the probe 21 maintains the linear state by its own rigidity, in other words, the distal end portion 22 of the probe 21 moves on a straight line. The endoscope system 1B performs calibration at a place where the distal end portion 22 moves on a straight line after projecting from the projection port 14B.

That is, the first calculation step is performed in the state shown in Fig. 10, the probe 21 moves by an amount for maintaining the linear state in the probe moving step, performs the second calculation step in the state shown in Fig. 11, and the navigation unit 31 thereby sets the axial direction of the probe 21 at an arbitrary time t, that is, the longitudinal direction VV(t) of the distal end portion 22 as a function of the magnetic field detection direction data V(t) from the coil 40A of the first sensor 40 and magnetic field detection direction data W(t) from the coil 40B.

That is, the endoscope system 1B of the present embodiment and the endoscope system 1 of the first embodiment only differ in the place where calibration is performed, but are basically the same in the system configuration and calibration method.

Even in the case of a side-viewing endoscope or oblique-viewing endoscope having a structure in which the probe 21 projects in the diagonal direction as in the case of the endoscope 11 B, the endoscope system 1 B of the present embodiment can obtain effects similar to those of the endoscope system 1B of the first embodiment.

<Third embodiment>

Hereinafter, an endoscope system 1C which is a medical equipment system according to a third embodiment of the present invention will be described with reference to the accompanying drawings. The endoscope system 1C of the present embodiment is similar to the endoscope system 1 of the first embodiment, and therefore the same components will be assigned the same reference numerals and descriptions thereof will be omitted.

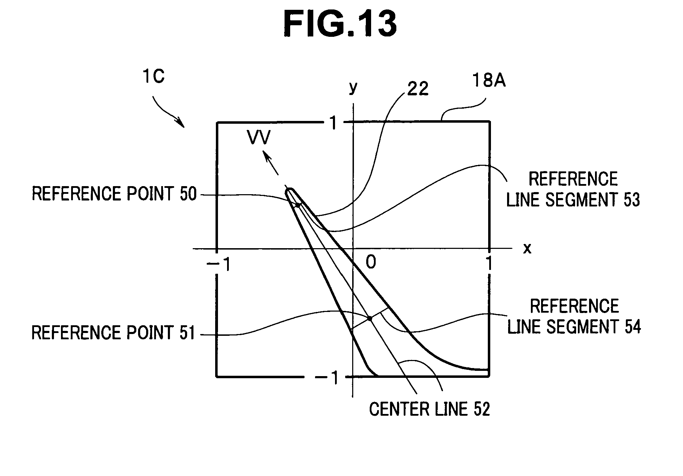

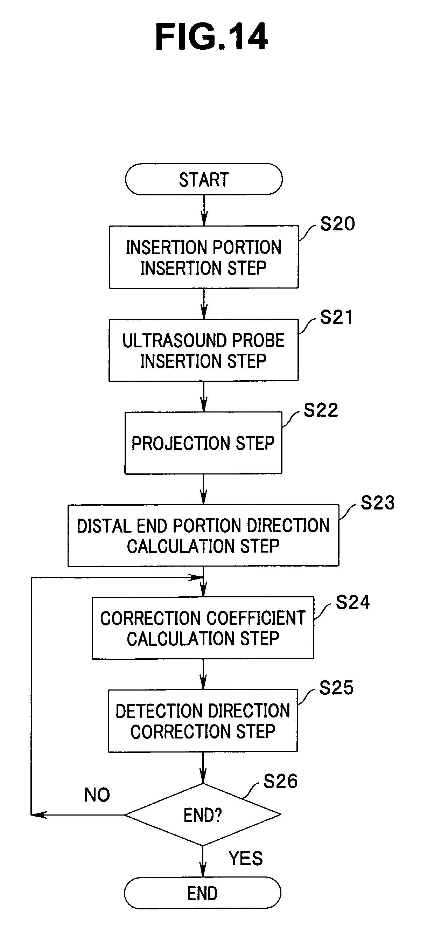

Fig. 13 is a display screen illustrating an example of image processing of the monitor 18 for illustrating the endoscope system of the third embodiment and Fig. 14 is a flowchart illustrating a processing flow of the endoscope system of the third embodiment.

In the endoscope system 1C, as shown in Fig. 13, the direction calculation section 31B in the navigation unit 31 projects from the projection port 14B and calculates the longitudinal direction of the distal end portion 22 through image processing based on an image of the probe 21 in the endoscope image 18A picked up by the CCD 19. Fig. 13 shows an example where the probe 21 is bent by gravity.

A direction calculation section 31BA which is different from the direction calculation section 31B of the first embodiment calculates the longitudinal direction of the distal end portion 22 of the probe 21 with respect to the direction of the second sensor 41 based on the shape of the probe 21 in the endoscope image 18A first.

There are several methods thereof and two of those methods will be described. A first method will be described below first. According to the first method, the endoscope image 18A is preliminarily photographed with the probe 21 projected in various projection directions and projection lengths, the direction of the distal end 22A of the probe 21 with respect to the direction of the second sensor 41 at that time is physically measured and a database is created according to the following procedure. The portion corresponding to the probe 21 and the other portion in each endoscope image 18A are identified, binarized and a binarized reference endoscope image is thereby created. At the time of photographing an endoscope image, the binarized reference endoscope image and the measured distal end direction of the probe 21 are associated with each other and saved, and a database is thereby created.

During use, an outer edge shape of the probe 21 is extracted from the current endoscope image 18A. The positions and shapes of the probe 21 are compared using the outer edge shape of the probe 21 extracted from the endoscope image 18A, a plurality of binarized reference endoscope images saved in the database and the endoscope image, a binarized reference endoscope image that best matches the position and shape of the probe 21 of the current endoscope image 18A is selected. The longitudinal direction of the distal end portion 22 associated with the selected binarized reference ultrasound image is assumed to be the longitudinal direction of the distal end portion 22 corresponding to the direction of the current second sensor 41.

Next, the second method will be described. In the second method, the outer edge shape of the probe 21 is extracted from the current endoscope image 18A during use. As shown in Fig. 13, a center line 52 is calculated on a longitudinal axis of the outer edge shape of the distal end portion 22 of the extracted probe 21 and two reference points 50 and 51 are set on the center line 52. Furthermore, reference line segments 53 and 54 which pass through reference points 50 and 51 and are orthogonal to the center line 52 are calculated. Here, a two-dimensional coordinate system is set assuming that the origin is the center position of the endoscope image 18A, the rightward direction is +x direction and the upward direction is +y direction. In this coordinate system, suppose the upper side of the endoscope image is y=1, the lower side is y=-1, the right side is x=1 and the left side is x=-1. Coordinates (x, y) of the two reference points in the coordinate system are calculated respectively.

Furthermore, lengths of the reference line segments 53 and 54 are calculated and assumed to be the values of z. Next, since the value of the angle of view which is a design value of the endoscope and the value of the outer diameter of the distal end portion 22 which is a design value of the probe 21 are known, it is possible to judge an approximate apparent outer diameter of the probe 21 on the endoscope image 18A in proportion to the distance between the probe 21 and the CCD 19. In other words, when the probe 21 is far from the CCD 19, its endoscope image 18A appears small and when the probe 21 is in the vicinity, its endoscope image 18A appears large. Thus, it is possible to calculate the distance between the CCD 19 in the three-dimensional space and the reference points 50 and 51 on the probe 21 from the values of z. On the other hand, it is possible to judge the direction of the (x, y) coordinates on the endoscope image 18A with respect to the CCD 19 in the three-dimensional space from the value of the angle of view which is a design value of the endoscope. To be exact, the (x, y) coordinate points on the endoscope image correspond to points on radial straight lines centered on the position of the CCD 19 in the three-dimensional space. From this, it is possible to calculate the directions of the reference points 50 and 51 on the probe 21 from the CCD 19 in the three-dimensional space from the (x, y) values. The positions of the reference points 50 and 51 of the probe 21 with respect to the CCD can be calculated from the distances between the CCD 19 and the reference points 50 and 51 on the probe 21 calculated from z described above, the directions of the reference points 50 and 51 on the probe 21 from the CCD 19 in the three-dimensional space calculated from (x, y).

Furthermore, the three-dimensional positional relationship between the CCD 19 and the second sensor 41 is known. For this reason, it is possible to convert the positions of the reference points 50 and 51 of the probe 21 with respect to the CCD 19 to positions of the reference points 50 and 51 of the probe 21 with respect to the second sensor 41 when assuming the position of the second sensor 41 is the origin and the directions of the second sensor 41 are x-, y- and z-axes. The direction of the vector connecting the two reference points on the probe 21 is the longitudinal direction of the distal end portion 22, and the longitudinal direction of the distal end portion 22 with respect to the direction of the second sensor 41 can be calculated.

Next, the direction calculation section 31 B converts the longitudinal direction of the distal end portion 22 with respect to the direction of the second sensor 41 to the longitudinal direction of the distal end portion 22 with respect to the direction of the first sensor 40. That is, the direction calculation section 31B performs coordinate transformation from the detection value of the first sensor 40 and the detection value of the second sensor 41 using the relationship between the position and direction of the first sensor 40 and the position and direction of the second sensor 41.

Next, a processing flow of the endoscope system 1C of the present embodiment will be described according to the flowchart in Fig. 14.

<Steps S20 and S21>

These are the same as steps S10 and S 11 in the description of the endoscope system 1 according to the first embodiment.

<Step S22> Projection step

The operator causes the probe 21 to project from the projection port 14B up to a sufficiently recognizable position in the endoscope image 18A as shown in Fig. 13.

<Step S23> Distal end portion direction calculation step

The navigation unit 31 performs image analysis of the state of the probe 21 in the endoscope image 18A using the aforementioned method and thereby calculates the longitudinal direction VV of the distal end portion 22 of the probe 21 with respect to the second sensor 41. In this case, VV is calculated using the direction of the second sensor 41 as shown in (Equation 9) as a reference.

The navigation unit 31 acquires direction data of the second sensor 41 simultaneously with the distal end portion direction calculation step. Of the direction data of the second sensor in this case, the longitudinal direction data of the distal end portion 22 (rigid portion 13) is assumed as a vector V6 and the direction data on the endoscope image 18A is assumed as a vector W6.

$$VV = a_4 V6 + b_4 W6 + c_4 X6 \qquad \text{(Equation 9)}$$

where

$$X6 = V6 \times W6 \text{ (vector product)} \qquad \text{(Equation 10)}$$

<Step S24> Correction coefficient calculation step

The navigation unit 31 acquires magnetic field detection direction data of the first sensor 40 simultaneously with the distal end portion direction calculation step. Suppose magnetic field detection direction data of the coil 40A of the first sensor 40 is a vector V5 and the magnetic field detection direction data of the coil 40B is a vector W5 in this case.

VV is expressed as a function of V5, W5 and X5 as (Equation 11) below. Relative position coefficients $a_5$, $b_5$ and $c_5$ can be calculated from VV calculated according to (Equation 9) and the detected values of V5, W5 and X5.

$$VV = a_5 V5 + b_5 W5 + c_5 X5 \qquad \text{(Equation 11)}$$

<Step S25> Detection direction correction step

The detection direction correction step of the endoscope system 1C is the same as the detection direction correction step S16 of the endoscope system 1 of the first embodiment.

The endoscope system 1C of the present embodiment has the effects of the endoscope system 1 of the first embodiment and can further detect the longitudinal direction of the distal end portion 22 of the probe accurately even when the probe 21 is bent due to influences of gravity or the like.

<Fourth embodiment>

Hereinafter, an endoscope system 1D according to a fourth embodiment will be described with reference to the accompanying drawings. The endoscope system 1D of the present embodiment is similar to the endoscope system 1 of the first embodiment, and therefore the same components will be assigned the same reference numerals and descriptions thereof will be omitted.

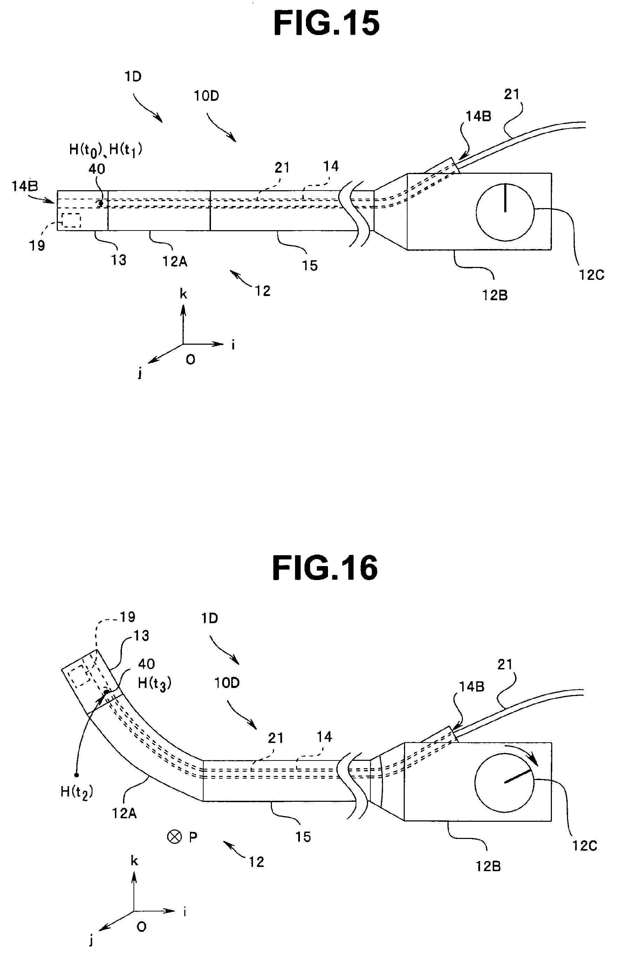

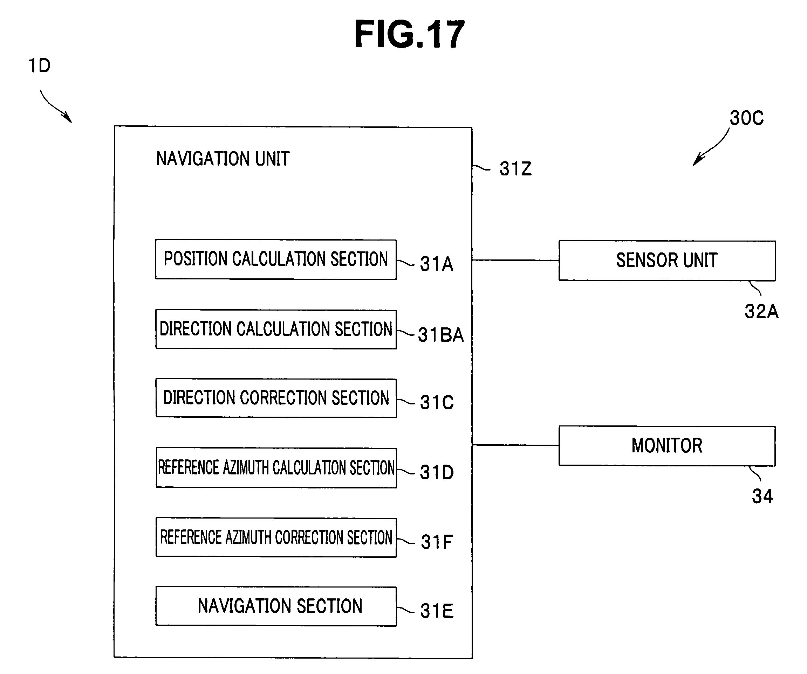

Fig. 15 and Fig. 16 are schematic cross-sectional views of the endoscope illustrating the endoscope system of the present embodiment and Fig. 17 is a configuration diagram illustrating a configuration of a navigation unit of the endoscope system of the present embodiment.

In navigation, it is important to accurately detect the longitudinal direction of the distal end portion 22 of the medical instrument of a small diameter to be made to project from the insertion portion of the endoscope as in the case of the endoscope system 1 of the first embodiment, and at the same time, it is also important to accurately detect a reference azimuth which is a predetermined azimuth within a plane (radial direction) perpendicular to the longitudinal direction. When, for example, the medical instrument is an ultrasound probe which radially scans a plane perpendicular to the longitudinal axis of the probe, detecting the vertical and horizontal directions within the scanning plane of the ultrasound transducer is important in judging the position of a lesioned region. Furthermore, when the medical instrument is forceps, it is important and necessary that the opening/closing direction of the forceps match the direction of the lesioned region.

Therefore, when, for example, a sensor made up of two coils, directions of coil axes of which are orthogonal to each other, is disposed at the distal end portion 22 of the ultrasound probe 21, it is ideal to ensure that the magnetic field detection direction of one coil be parallel to the longitudinal direction of the distal end portion 22 of the ultrasound probe 21 and the magnetic field detection direction of the other coil be parallel to the reference azimuth (e.g., upward direction of the ultrasound image).

However, as has already been described, it is not easy to dispose on the distal end portion 22 of the probe 21 of an extremely small diameter, a two-axis magnetic field sensor of a still smaller diameter so that one detection axis thereof is parallel to the longitudinal direction of the distal end portion 22 and the other detection axis is parallel to the upward direction of the ultrasound image. Thus, as shown in Fig. 4, the coil axis direction which is the magnetic field detection direction of the coil 40B may not be completely parallel to the reference azimuth. The operator cannot accurately grasp the vertical and horizontal directions of the ultrasound image.

For this reason, the endoscope system 1D detects variations in the position and direction of the sensor 40 due to a rotation operation of the probe 21 and the direction calculation section 31 B calculates the exact longitudinal direction of the distal end portion 22. On the other hand, variations in the position and direction of the sensor 40 due to a bending operation of the bending portion 12A of the probe 21 (see Fig. 15) are detected and the reference azimuth calculation section 31D (see Fig. 16) which is reference azimuth calculation means calculates a precise reference azimuth. That is, the endoscope system 1D calculates a distal end direction correction value for correcting the direction of the sensor 40 to the distal end longitudinal direction of the probe 21 through calibration by the rotation operation of the probe 21 and calculates a reference azimuth correction value for correcting the direction of the sensor 40 to a reference azimuth through calibration by a bending operation.

As shown in Fig. 15, the endoscope 11D of the endoscope system 1D of the present embodiment includes a bending portion 12A disposed between the flexible portion 15 and the rigid portion 13 of the insertion portion 12. Furthermore, image pickup means such as a CCD 13B is disposed in the rigid portion 13 and the operator can recognize an endoscope image picked up by the CCD 13B and displayed on the monitor 18. The bending portion 12A is connected to a bending knob 12C of an operation portion 12B via a bending wire (not shown). As shown in Fig. 16, when the operator rotates the bending knob 12C, the bending portion 12A performs bending operation and the distal end 13A of the insertion portion 12 performs rotational motion.

As shown in Fig. 17, in the endoscope system 1D of the present embodiment, the navigation unit 31 Z includes a reference azimuth calculation section 31D that calculates a reference azimuth of an ultrasound image picked up by the ultrasound transducer 23 based on the positions before and after movement of the first sensor 40 by the rotation operation of the probe 21 and the bending operation of the bending portion 12A.

Next, sections in the navigation unit 3 1 Z of the endoscope system 1D of the present embodiment will be described. Since the position calculation section 31A is the same as that of the first embodiment, the direction calculation section 31B will be described first.

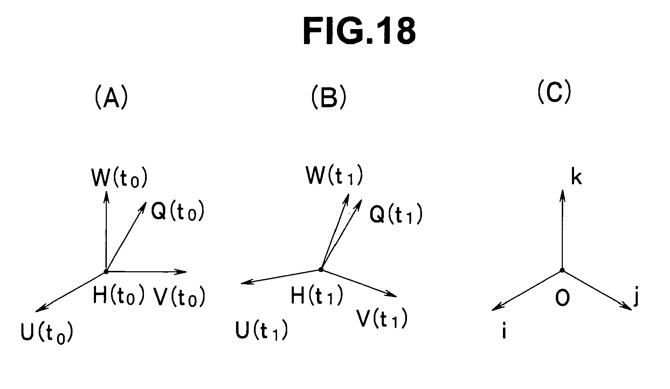

Fig. 18A to Fig. 18C are diagrams for illustrating a coordinate system in a rotation operation of the probe 21 of the endoscope system 1D of the present embodiment. Suppose a position of the first sensor 40 in a state (time to) before the rotation operation of the probe 21 is $H(t_0)$ and an orthonormal basis in the direction of the first sensor 40 is $(U(t_0)V(t_0)W(t_0))$ as shown in Fig. 18A, a position of the first sensor 40 in a state (time $t_1$) after the rotation operation of the probe 21 is $H(t_1)$ and an orthonormal basis in the direction of the first sensor 40 is $(U(t_1)V(t_1)W(t_1))$ as shown in Fig. 18B, and an orthonormal basis provided in the center of the transmission antenna 33 is (ijk) as shown in Fig. 18C.

First, the operator twists the probe 21 in the channel 14 in such a way that the bending portion 12A as shown in Fig. 15 is not bent, that is, is straight, in other words, rotates the probe 21 around the center direction of its longitudinal axis. The direction of the axis of rotation of the probe 21 is a distal end direction Q of the probe 21. Since the rotation operation is an operation for calculating the axis of rotation from a state variation before and after the rotation, the rotation operation may be a half turn or so.

$(U(t_0), V(t_0), W(t_0))$ and $(U(t_1), V(t_1), W(t_1))$ can be expressed using matrices $S(t_0)$ and $S(t_1)$ of three rows and three columns respectively as follows. The respective components of $S(t_0)$ and $S(t_1)$ are successively outputted from the sensor unit 32.

$$[i(t_0)j(t_0)k(t_0)]=[U(t_0)V(t_0)W(t_0)]S(t_0) \qquad \text{(Equation 12)}$$

$$[i(t_1)j(t_1)k(t_1)]=[U(t_1)V(t_1)W(t_1)]S(t_1) \qquad \text{(Equation 13)}$$

Here, $S(t_0)$ and $S(t_1)$ can be expressed as (Equation 14) and (Equation 15) below using row vectors $S_1$, $S_2$ and $S_3$ of three elements shown below. where,

$$s_1=(s_{11} \ s_{12} \ s_{13})$$

$$s_2=(s_{21} \ s_{22} \ s_{23})$$

$$s_3=(s_{31} \ s_{32} \ s_{33})$$

(Equation 14)

$$S(t_0) = \begin{pmatrix} s_1(t_0) \\ s_2(t_0) \\ s_3(t_0) \end{pmatrix}$$

(Equation 15)

$$S(t_1) = \begin{pmatrix} s_1(t_1) \\ s_2(t_1) \\ s_3(t_1) \end{pmatrix}$$

According to (Equation 12), since $S(t_0)$ is an orthogonal matrix, $[U(t_0)V(t_0)W(t_0)]$ can be expressed by (Equation 16). Here, symbol "T" affixed at the top left of each matrix means that the matrix is transformed into a transposed matrix.

(Equation 16)

$$[U(t_0)V(t_0)W(t_0)] = [i(t_0)j(t_0)k(t_0)]^T S(t_0)$$

$$= [i(t_0)j(t_0)k(t_0)] \begin{pmatrix} s_{11}(t_0) & s_{21}(t_0) & s_{31}(t_0) \\ s_{12}(t_0) & s_{22}(t_0) & s_{32}(t_0) \\ s_{13}(t_0) & s_{23}(t_0) & s_{33}(t_0) \end{pmatrix}$$

$$= [i(t_0)j(t_0)k(t_0)] [\,^T s_1(t_0)\,^T s_2(t_0)\,^T s_3(t_0)\,]$$

According to (Equation 13), since $S(t_1)$ is an orthogonal matrix, $[U(t_1)V(t_1)W(t_1)]$ can be expressed by (Equation 17).

(Equation 17)

$$[U(t_1)V(t_1)W(t_1)] = [i(t_1)j(t_1)k(t_1)]^T S(t_1)$$

$$= [i(t_1)j(t_1)k(t_1)] \begin{pmatrix} s_{11}(t_1) & s_{21}(t_1) & s_{31}(t_1) \\ s_{12}(t_1) & s_{22}(t_1) & s_{32}(t_1) \\ s_{13}(t_1) & s_{23}(t_1) & s_{33}(t_1) \end{pmatrix}$$

$$= [i(t_1)j(t_1)k(t_1)] [\,^T s_1(t_1)\,^T s_2(t_1)\,^T s_3(t_1)\,]$$

On the other hand, assuming the respective azimuth components corresponding to (ijk) of $U(t_0)$, $V(t_0)$, $W(t_0)$,

$U(t_1)$, $V(t_1)$ and $W(t_1)$ are column vectors $u(t_0)$, $v(t_0)$, $w(t_0)$, $u(t_1)$, $v(t_1)$ and $w(t_1)$ of three elements, the following (Equation 18) and (Equation 19) hold.

$$[U(t_0)V(t_0)W(t_0)]= [i(t_0)j(t_0)k(t_0)][u(t_0)v(t_0)w(t_0)] \quad \text{(Equation 18)}$$

$$[U(t_1)V(t_1)W(t_1)]=[i(t_1)j(t_1)k(t_1)][u(t_1)v(t_1)w(t_1)] \quad \text{(Equation 19)}$$

Since [i, j, k] are orthonormal bases, the following (Equation 20) is obtained from (Equation 16) and (Equation 18).

$$u(t_0)={}^{T}s_1(t_0), \ v(t_0)={}^{T}s_2(t_0), \ w(t_0)={}^{T}s_3(t_0) \quad \text{(Equation 20)}$$

Likewise, the following (Equation 21) is obtained from (Equation 17) and (Equation 19).

$$u(t_1)= {}^{T}s_1(t_1), \ v(t_1)={}^{T}s_2(t_1), \ w(t_1)={}^{T}s_3(t_1) \quad \text{(Equation 21)}$$

The distal end direction Q is invariable before and after the rotation, that is, independent of time. Therefore, the following (Equation 22), (Equation 23) and (Equation 24) hold.

$$U(t_0)\cdot Q=U(t_1)\cdot Q \quad \text{(Equation 22)}$$

$$V(t_0)\cdot Q=V(t_1)\cdot Q \quad \text{(Equation 23)}$$

$$W(t_0)\cdot Q=W(t_1)\cdot Q \quad \text{(Equation 24)}$$

Here, assuming the matrix whose elements are the respective direction components corresponding to [ijk] of Q is q, the following (Equation 25), (Equation 26) and (Equation 27) hold.

$$0=U(t_0)\cdot Q-U(t_1)\cdot Q=(U(t_0)-U(t_1))\cdot Q={}^{T}(u(t_0)-u(t_1))q={}^{T}({}^{T}s_1(t_0)-{}^{T}s_1(t_1))q=(s_1(t_0)-s_1(t_1))q \quad \text{(Equation 25)}$$

$$0=V(t_0)\cdot Q-V(t_1)\cdot Q=(V(t_0)-V(t_1))\cdot Q={}^{T}(v(t_0)-v(t_1))q={}^{T}({}^{T}s_2(t_0)-{}^{T}s_2(t_1))q=(s_2(t_0)-s_2(t_1))q \quad \text{(Equation 26)}$$

$$0=W(t_0)\cdot Q-W(t_1)\cdot Q=(W(t_0)-W(t_1))\cdot Q={}^{T}(w(t_0)-w(t_1))q={}^{T}({}^{T}s_3(t_0)-{}^{T}s_3(t_1))q=(s_3(t_0)-s_3(t_1))q \quad \text{(Equation 27)}$$

where,

(Equation 28)

$$\begin{pmatrix} s_1(t_0) - s_1(t_1) \\ s_2(t_0) - s_2(t_1) \\ s_3(t_0) - s_3(t_1) \end{pmatrix} q = 0$$

That is,

$$(S(t_0)-S(t_1))q=0 \qquad \text{(Equation 29)}$$

Q is the axis of rotation and since the position H of the sensor 40 moves within a plane perpendicular to the axis of rotation during rotation, the following (Equation 30) holds.

$$Q\cdot(H(t_0)-H(t_1))=0 \qquad \text{(Equation 30)}$$

That is, assuming matrices whose elements are the respective direction components corresponding to {ijk} of $H(t_0)$ and $H(t_1)$ are $h(t_0)$ and $h(t_1)$, the following (Equation 31) holds. The respective components of $H(t_0)$ and $H(t_1)$ are outputted from the sensor unit 32.

$$^T(h(t_1)-h(t_0))q=0 \qquad \text{(Equation 31)}$$

Furthermore, since the Q is a basic vector, the following (Equation 32) holds.

$$|q|=1 \qquad \text{(Equation 32)}$$

Therefore, the q is calculated from (Equation 29), (Equation 31) and (Equation 32).
When the calculated Q is expressed as a function of $U(t_1)$, $V(t_1)$ and $W(t_1)$, the Q is expressed by the following (Equation 33) and relative position coefficients $a_6$, $b_6$ and $c_6$ are calculated.

$$Q=a_6U(t_1)+b_6V(t_1)+c_6W(t_1) \qquad \text{(Equation 33)}$$

Next, the operation of the direction correction section 31C will be described. The operation of the direction correction section 31C is basically the same as the operation of the first embodiment, that is, corrects the direction of the first sensor 40 and successively calculates the longitudinal direction of the distal end portion 22. To be more specific, the direction correction section 31C operates as follows.
Assuming the direction of the first sensor 40 at an arbitrary time t is U(t), V(t) and W(t), the navigation unit 31 can calculate the distal end direction Q(t) of the probe from the following (Equation 34).

$$Q(t)=a_6U(t)+b_6V(t)+c_6W(t) \qquad \text{(Equation 34)}$$

The Q(t) calculated here is transmitted to the navigation section 31E.
Furthermore, the operation of the reference azimuth calculation section 31D will be described.
The operator bends the bending portion 12A in an upward direction of the ultrasound image using the bending knob 12C as shown in Fig. 16. Suppose the axis of rotation of the bending operation in this case is P. Moreover, suppose time before the bending operation is $t_2$ and time after the bending operation is $t_3$. Using a method similar to the above described method of calculating the Q, the direction P of the bending axis of rotation when performing a bending operation is calculated.

When the calculated P is expressed as a function of $U(t_3)$, $V(t_3)$ and $W(t_3)$, the P is expressed as (Equation 35) below, and the reference azimuth calculation section 31D can calculate relative position functions $a_7$, $b_7$ and $c_7$ according to (Equation 35).

$$P=a_7U(t_3)+b_7V(t_3)+c_7W(t_3) \qquad \text{(Equation 35)}$$

Furthermore, the reference azimuth calculation section 31D which is reference azimuth calculation means calculates the reference azimuth $V_{12}(t_3)$ according to the following (Equation 36).

$$V_{12}(t_3)=P\times Q(t_3)=(b_7c_6-c_7b_6)U(t_3)+(c_7a_6-a_7c_6)V(t_3)+(b_7c_6-c_7b_6)W(t_3)$$

$$\text{(Equation 36)}$$

Next, the operation of the reference azimuth correction section 31F which is the reference azimuth correction means will be described. The reference azimuth correction section 31F corrects the direction of the first sensor 40 and successively calculates the reference azimuth. To be more specific, the reference azimuth correction section 31F operates as follows.

When the direction of the first sensor 40 at an arbitrary time t is assumed to be U(t), V(t) and W(t), the navigation unit 31 calculates the reference azimuth $V_{12}(t)$ of the probe according to the following (Equation 37).

$$V_{12}(t)=(b_7c_6-c_7b_6)U(t)+(c_7a_6-a_7c_6)V(t)+(b_7c_6-c_7b_6)W(t) \qquad \text{(Equation 37)}$$

The Q(t) calculated here is transmitted to the navigation section 31E.

Finally, the operation of the navigation section will be described. The navigation section performs navigation based on the Q(t) calculated by the direction correction section and the $V_{12}(t)$ calculated by the reference azimuth correction section 31F.

As described above, the endoscope system 1D corrects the direction of the first sensor 40 to the distal end direction of the probe 21 through calibration by a rotation operation of the probe 21 and corrects the direction of the first sensor 40 to the reference azimuth through calibration by a bending operation. Thus, the operator can accurately grasp the vertical and horizontal directions of an ultrasound image and perform inspection or treatment with high accuracy.

When the direction of the first sensor 40 and the upward direction of the ultrasound image are matched through calibration by the bending operation, if the probe is provided with a bending mechanism, the operator may perform a bending operation of the probe.

An ultrasound probe has been described above as an example of medical instrument of the medical equipment system and an upward direction of the endoscope image has been described above as a reference method, but in the case where the medical instrument is forceps, the opening/closing direction of the forceps is set as the reference azimuth. Furthermore, in the case where the medical instrument is a single-edged knife, the direction of the edge is set as the reference azimuth. Furthermore, when the medical instrument is a small endoscope inserted into the channel of the endoscope, the upward direction of an endoscope image of the small endoscope is set as the reference azimuth.

The distal end direction Q is calculated above from (Equation 29), (Equation 31) and (Equation 32), but when the value of H in (Equation 30) has substantially no difference between times to and $t_1$, the error of the distal end direction Q increases. For this reason, when the distance between $H(t_0)$ and $H(t_1)$ is equal to or below a predetermined value, the medical equipment system preferably displays a message on the screen of the monitor 18 and instruct the operator to further rotate the probe 21.

The calculation in this case is as follows. Assuming the time after a second rotation is t4, the following (Equation 38) holds in the same way as (Equation 29).

$$(S(t_0)-S(t_4))q=0 \qquad \text{(Equation 38)}$$

The distal end direction Q of the distal end portion 22 is calculated from (Equation 29), (Equation 32) and (Equation 38). In this way, the error becomes smaller.

Furthermore, as in the cases of the first to third embodiments, the second sensor may be provided at the endoscope distal end and the position and direction information of the probe may be corrected based on information of the second sensor.

The present invention is not limited to the aforementioned embodiments, but various changes, modifications or the like can be made without departing from the spirit and scope of the present invention.

For example, the detection means for detecting the position and direction may not necessarily be a magnetic sensor. For example, a gyro sensor may be disposed at the distal end portion to detect the position and direction, a light-emitting marker such as LED may be disposed at the operation portion of a rigid endoscope, the light-receiving apparatus may detect the position and direction of the operation portion of the endoscope, convert the position to the position of the endoscope distal end portion or a fiber grating (FBG) sensor may be disposed at the insertion portion of the endoscope to detect the position and direction of the distal end portion.

Furthermore, although the flexible endoscope having the flexible portion 15 and the rigid portion 13 disposed on the distal end side of the flexible portion 15 has been described above as an example of the insertion means of the medical equipment system, the present invention is not limited to this but the insertion means may be a rigid endoscope, trocar or the like as long as the insertion means has a channel.

That is, the probe is moved in the channel of the endoscope above to correct the probe direction, but in an endoscope operation, the endoscope or treatment instrument may be moved in the trocar to correct the direction of the endoscope or treatment instrument.

As described above, the endoscope system 1D is as follows.

(1) A medical equipment system including:

insertion means including a flexible portion, a bending portion, a rigid portion and a channel that passes through the flexible portion, the bending portion and the rigid portion;

a medical instrument that is inserted from an insertion port on a proximal end portion side of the channel, projects from a projection port of the rigid portion and includes a sensor for detecting a position and direction at a distal end portion;

position calculation means for calculating the position and direction of the distal end portion from information of the sensor; and

reference azimuth calculation means for calculating, when the medical instrument rotates in the channel, a reference azimuth of the distal end portion based on the position and direction of the distal end portion before and after movement when the bending portion is bent.

(2) The medical equipment system described in (1) above, wherein the insertion means is an insertion portion of an endoscope, and

the reference azimuth is an azimuth of an image picked up by the endoscope.

(3) The medical equipment system described in (1) above, wherein the medical instrument is an ultrasound probe, and the reference azimuth is an azimuth of an ultrasound image picked up by the ultrasound probe.

The present application is filed claiming the priority of Japanese Patent Application No. 2009-132390 filed in Japan on June 1, 2009, and the above described disclosure is incorporated by reference in the present description, claims and drawings.

## Claims

1. A medical equipment system comprising:

insertion means having a rigid portion configuring a distal end portion of an insertion portion and a channel passed through inside of the rigid portion;

a medical instrument whose medical instrument distal end portion projects from a projection port of the rigid portion, the medical instrument being inserted from an insertion port on a proximal end portion side of the channel; and
direction calculation means that calculates a longitudinal direction of the medical instrument distal end portion based on a positional variation of the medical instrument distal end portion inserted into the channel.

2. The medical equipment system according to claim 1, wherein the direction calculation means calculates the longitudinal direction based on a positional variation caused by linear movement of the medical instrument distal end portion.

3. The medical equipment system according to claim 2, further comprising:

a first sensor disposed at the medical instrument distal end portion for detecting a position and a direction; and
position calculation means that calculates a position and a direction of the first sensor,
wherein the direction calculation means calculates a longitudinal direction of the medical instrument distal end portion based on the information calculated by the position calculation means.

4. The medical equipment system according to claim 3, wherein the insertion means comprises a second sensor disposed in the rigid portion for detecting a position and a direction, and
the direction calculation means calculates a longitudinal direction of the medical instrument distal end portion based on information of the first sensor and the second sensor.

5. The medical equipment system according to claim 4, wherein the first sensor and the second sensor are sensors that detect a magnetic field of at least two axial directions respectively, and
the medical equipment system further comprises magnetic field generation means that generates a magnetic field for the first sensor and the second sensor to detect the position and direction.

6. The medical equipment system according to claim 1, wherein the insertion means comprises a bending portion on a proximal end portion side of the rigid portion,
the medical instrument comprises a first sensor that detects a position and a direction at the medical instrument distal end portion,
the medical equipment system further comprises a reference azimuth calculation section that calculates a reference azimuth within a plane orthogonal to a longitudinal direction of the medical instrument distal end portion based on information of the first sensor when the bending portion is bent, and
the direction calculation means calculates a longitudinal direction of the medical instrument distal end portion based on a position variation when the first sensor rotates inside the channel.

7. The medical equipment system according to claim 1, wherein the insertion means is an endoscope apparatus comprising image pickup means disposed at the rigid portion, and
the direction calculation means calculates a longitudinal direction of the medical instrument distal end portion which projects from a projection port of the rigid portion based on an image picked up by the image pickup means.

8. The medical equipment system according to any one of claims 1 to 7, wherein the insertion means is an endoscope apparatus comprising image pickup means disposed at the rigid portion, and
the medical instrument is a treatment instrument or an ultrasound probe comprising an ultrasound transducer at the medical instrument distal end portion.

9. The medical equipment system according to claim 8, wherein the insertion supporting means comprises navigation means that performs navigation of inserting the medical instrument distal end portion into a region where images cannot be picked up by the image pickup means, based on a position of the medical instrument distal end portion.

10. A medical instrument calibration method, comprising:

an insertion step of inserting the medical instrument from an insertion port on a proximal end portion side of a channel of insertion means having a rigid portion configuring a distal end portion of an insertion portion and the channel passed through inside of the rigid portion;
a first calculation step of calculating the position of the medical instrument distal end portion in a first place based on information of a first sensor disposed at the medical instrument distal end portion, capable of detecting a position and direction;
a probe moving step of moving the position of the medical instrument distal end portion from the first place to a second place on a straight line;

a second calculation step of calculating the position of the medical instrument distal end portion in the second place; and

a distal end portion direction calculation step of calculating the longitudinal direction of the medical instrument distal end portion based on the position calculated in the first calculation step and the position calculated in the second calculation step.

11. The medical instrument calibration method according to claim 10, wherein the first place and the second place are inside the channel of the rigid portion.

12. The medical instrument calibration method according to claim 11, wherein the insertion means is an endoscope apparatus comprising image pickup means disposed at the rigid portion.

13. The medical instrument calibration method according to claim 12,
wherein the insertion means comprises a second sensor that detects a position in the rigid portion, and
the method further comprises a position correction step of correcting the position of the medical instrument distal end portion based on information of the second sensor.

14. The medical instrument calibration method according to claim 13, wherein the medical instrument is a treatment instrument or an ultrasound probe comprising an ultrasound transducer at the medical instrument distal end portion.

# FIG.1

FIG.2

EP 2 335 553 A1

# FIG.3

# FIG.4

# FIG.5

NAVIGATION UNIT ~31

POSITION CALCULATION SECTION ~31A

DIRECTION CALCULATION SECTION ~31B

DIRECTION CORRECTION SECTION ~31C

NAVIGATION SECTION ~31E

SENSOR UNIT 32

MONITOR 34

30

EP 2 335 553 A1

# FIG.6

```
                  ┌─────────────┐
                  │    START    │
                  └──────┬──────┘
                         │
          ┌──────────────▼──────────────┐   S10
          │    INSERTION PORTION         │
          │    INSERTION STEP            │
          └──────────────┬──────────────┘
                         │
          ┌──────────────▼──────────────┐   S11
          │    ULTRASOUND PROBE          │
          │    INSERTION STEP            │
          └──────────────┬──────────────┘
                         │
          ┌──────────────▼──────────────┐   S12
          │    FIRST CALCULATION         │
          │    STEP                      │
          └──────────────┬──────────────┘
                         │
          ┌──────────────▼──────────────┐   S13
          │    PROBE MOVING              │
          │    STEP                      │
          └──────────────┬──────────────┘
                         │
          ┌──────────────▼──────────────┐   S14
          │    SECOND CALCULATION        │
          │    STEP                      │
          └──────────────┬──────────────┘
                         │
          ┌──────────────▼──────────────┐   S15
          │  CORRECTION COEFFICIENT      │
          │  CALCULATION STEP            │
          └──────────────┬──────────────┘
                         │
          ┌──────────────▼──────────────┐   S16
          │  DETECTION DIRECTION         │
          │  CORRECTION STEP             │
          └──────────────┬──────────────┘
                         │
                 NO   ◇──▼──◇   S17
              ◄───────│ END? │
                      ◇──┬──◇
                         │ YES
                  ┌──────▼──────┐
                  │    END      │
                  └─────────────┘
```

# FIG.7

# FIG.8

# FIG.9

# FIG.10

# FIG.11

# FIG.12

# FIG.13

# FIG.14

```
            ┌─────────────┐
            │    START    │
            └─────────────┘
                   │
                   ▼
        ┌──────────────────────┐  ╱ S20
        │  INSERTION PORTION   │
        │   INSERTION STEP     │
        └──────────────────────┘
                   │
                   ▼
        ┌──────────────────────┐  ╱ S21
        │   ULTRASOUND PROBE   │
        │   INSERTION STEP     │
        └──────────────────────┘
                   │
                   ▼
        ┌──────────────────────┐  ╱ S22
        │   PROJECTION STEP    │
        └──────────────────────┘
                   │
                   ▼
    ┌──────────────────────────────┐  ╱ S23
    │  DISTAL END PORTION DIRECTION │
    │      CALCULATION STEP         │
    └──────────────────────────────┘
                   │
         ┌─────────┤
         │         ▼
         │ ┌──────────────────────┐  ╱ S24
         │ │ CORRECTION COEFFICIENT│
         │ │   CALCULATION STEP    │
         │ └──────────────────────┘
         │         │
         │         ▼
         │ ┌──────────────────────┐  ╱ S25
         │ │  DETECTION DIRECTION  │
         │ │   CORRECTION STEP     │
         │ └──────────────────────┘
         │         │
         │         ▼           ╱ S26
   NO    │      ╱──────╲
  ◄──────┤     ╱  END?   ╲
         └────╲          ╱
               ╲────────╱
                   │ YES
                   ▼
            ┌─────────────┐
            │     END     │
            └─────────────┘
```

# FIG.15

# FIG.16

# FIG.17

EP 2 335 553 A1

1D

NAVIGATION UNIT — 31Z

POSITION CALCULATION SECTION — 31A

DIRECTION CALCULATION SECTION — 31BA

DIRECTION CORRECTION SECTION — 31C

REFERENCE AZIMUTH CALCULATION SECTION — 31D

REFERENCE AZIMUTH CORRECTION SECTION — 31F

NAVIGATION SECTION — 31E

30C

SENSOR UNIT

32A

MONITOR

34

# FIG.18

(A)

W($t_0$)

Q($t_0$)

U($t_0$)   H($t_0$)  V($t_0$)

(B)

W($t_1$)

Q($t_1$)

U($t_1$)   H($t_1$)

V($t_1$)

(C)

k

i   O   j

<table>
<tr><td colspan="2" align="center">**INTERNATIONAL SEARCH REPORT**</td><td>International application No.<br>PCT/JP2010/057456</td></tr>
</table>

A. CLASSIFICATION OF SUBJECT MATTER
*A61B1/00*(2006.01)i

According to International Patent Classification (IPC) or to both national classification and IPC

B. FIELDS SEARCHED

Minimum documentation searched (classification system followed by classification symbols)
A61B1/00

Documentation searched other than minimum documentation to the extent that such documents are included in the fields searched
| Jitsuyo Shinan Koho | 1922–1996 | Jitsuyo Shinan Toroku Koho | 1996–2010 |
| Kokai Jitsuyo Shinan Koho | 1971–2010 | Toroku Jitsuyo Shinan Koho | 1994–2010 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y<br>A | JP 2006-223849 A (Pentax Corp.),<br>31 August 2006 (31.08.2006),<br>entire text; fig. 1 to 3<br>(Family: none) | 1-5,7-14<br>6 |
| Y<br>A | JP 2003-524443 A (Super Dimension Ltd.),<br>19 August 2003 (19.08.2003),<br>entire text; fig. 1 to 16<br>& US 6593884 B1 & EP 2100557 A1<br>& WO 2000/010456 A1 | 1-5,8-14<br>6,7 |
| Y<br>A | JP 2005-40400 A (Olympus Corp.),<br>17 February 2005 (17.02.2005),<br>paragraphs [0039] to [0042]; fig. 14 to 15<br>(Family: none) | 7<br>1-6,8-14 |

☐ Further documents are listed in the continuation of Box C. ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>28 May, 2010 (28.05.10) | Date of mailing of the international search report<br>08 June, 2010 (08.06.10) |
|---|---|
| Name and mailing address of the ISA/<br>Japanese Patent Office | Authorized officer |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004180940 A
- JP 2006223849 A
- JP 2007130154 A
- JP 2009132390 A